Europäisches Patentamt

European Patent Office

Office européen des brevets

(19)

(11) Veröffentlichungsnummer: **0 275 336**

**A1**

## (12) EUROPÄISCHE PATENTANMELDUNG

(21) Anmeldenummer: **87100672.2**

(22) Anmeldetag: **20.01.87**

(51) Int. Cl.⁴ **A61K 9/22**

(43) Veröffentlichungstag der Anmeldung:
**27.07.88 Patentblatt 88/30**

(84) Benannte Vertragsstaaten:
**AT BE CH DE ES FR GB IT LI NL SE**

(71) Anmelder: **KNOLL AG**
**Knollstrasse**
**D-6700 Ludwigshafen(DE)**

(72) Erfinder: **Einig, Heinz, Dr.**
**Aspenweg 12**
**D-6730 Neustadt(DE)**

(74) Vertreter: **Karau, Wolfgang Dr. et al**
**BASF Aktiengesellschaft Carl-Bosch-Strasse 38**
**D-6700 Ludwigshafen(DE)**

(54) Arzneimittelzubereitung mit protahierender Wirkung.

(57) Die Erfindung betrifft eine feste Arzneimittelzubereitung auf Alginatbasis mit protrahierender Wirkung sowie Verfahren zu deren Herstellung.

EP 0 275 336 A1

## Arzneimittelzubereitung mit protrahierender Wirkung

Die Erfindung betrifft eine feste Arzneimittelzubereitung mit protrahierender Wirkung sowie Verfahren zu deren Herstellung.

Es ist bereits bekannt, Arzneimittelzubereitungen mit protrahierender Wirkung unter Verwendung von Alginaten herzustellen, vgl. Arzneim. Forsch. 25, 1272 (1975), DE-OS 20 62 715, Pharmazie 38, 473 (1983), Pharm. Sci 64, 166 (1975) und Pharm. Sci 65, 1170 (1976). Diese Zubereitungen sind jedoch noch nicht optimal hinsichtlich ihrer Freisetzungsdauer.

Es wurde nun gefunden, daß man die Arzneimittelzubereitungen auf Alginatbasis die wesentlich verbessern kann.

Gegenstand der Erfindung ist eine feste Arzneimittelzubereitung auf Alginatbasis mit protrahierender Wirkung, die dadurch gekennzeichnet ist, daß sie als Alginate eine Mischung aus einem Alkalialginat oder einem Polyethlenglykolalginat und einem Alkali-Erdalkali-Alginat enthält.

Als Alkalialginate kommen beispielsweise Ammoniumalginat, Kaliumalginat und vorzugsweise Natriumalginat in Betracht. Die Alkalialginate können durch Magnesiumalginat ersetzt sein.

Als Alkali-Erdalkali-Alginate eignen sich solche, die als Alkali Ammonium, Kalium, Magnesium und vorzugsweise Natrium und als Erdalkali Barium, Strontium und vorzugsweise Calcium enthalten. Die Erdalkalialginate können auch durch andere Alginate, z.B. Eisenalginat, ersetzt sein. Magnesiumalginat eignet sich nicht als Erdalkalialginat. Bevorzugtes Alkali-Erdalkali-Alginat ist das Natrium-Calcium-Alginat.

Die Alginate sind bekannte Verbindungen. Bevorzugt verwendet man solche, in denen das Verhältnis von Manuron zu Guluronsäure zwischen 3:7 und 7:3, vorzugweise 4:6 bis 6:4, liegt. Natriumalginat ist unter der Bezeichnung Texamid® und Natriumcalciumalginat unter der Bezeichung Kelset® im Handel erhältlich. Die Alkali-Erdalkali-Alginate kann man auch durch Mischen der einzelnen Alginate in feuchtem Zustand, Sieben und Trocknen herstellen. Sie enthalten das Alkali-und Erdalkali-Alginat vorzugsweise im Verhältnis von1:0,4 bis 1:2.

In der Mischug Alkalialginat/Alkali-Erdalkali-Alginat liegen die beiden Komponenten vorzugsweise im Bereich von 10:1 bis 1:1 vor. Das günstigste Verhältnis muß für jeden Wirkstoff einzeln ermittelt werden.

1 %ige wäßrige Lösungen der verwendeten Alginate sollten eine Viskosität von 70 bis 210 mPa.sec, vorzugsweise 80 bis 120 mPa.sec, besitzen.

Neben den Wirkstoffen und Alginaten enthalten die festen Arzneimittelzubereitungen übliche galenische Hilfsstoffe, wie Tablettenbinder, Füllstoffe, Konservierungsmittel, Fließreguliermittel, Weichmacher, Netzmittel und/oder Antioxidantien. Die Arzneimittelzubereitungen können auch mit einem magensaftlöslichen Überzug versehen sein (vgl. H. Sucker et al: Pharmazeutische Technologie, Thieme-Verlag, Stuttgart 1978). Am besten eignen sich als Zubereitungsformen Tabletten sowie Kapseln.

Als Wirkstoffe eignen sich für die Zubereitungen beispielsweise Verapamil, Soquinolol (DE-PS 2 519 163), Biperiden, Hydromorphon und insbesondere Gallopamil und Propranolol.

Die erfindungsgemäße Arzneimittelzubereitung hat den Vorteil, daß der Wirkstoff sehr konstant und über einen langen Zeitraum freigesetzt wird. Dadurch wird eine Therapie möglich, bei der das Arzneimittel mit sehr geringer Frequenz appliziert werden muß. Ein weiterer Vorteil ist der, daß die Zubereitung deutlich mehr als 50 % Wirkstoff enthalten kann. Darüber hinaus sind die erfindungsgemäßen Zubereitungen galenisch einfacher herstellbar als solche, die nur ein einziges Alginat enthalten.

Beispiel

Es wurden Tabletten folgender Zusammensetzung hergestellt:

|                        | I      | II     | III    | IV     |
|------------------------|--------|--------|--------|--------|
| Natriumalginat         | 230 mg | 180 mg | 130 mg | 120 mg |
| Natrium-Calcium-Alginat| 30 mg  | 80 mg  | 130 mg | 80 mg  |
| Polyvinylpyrrolidon    | 13 mg  | 13 mg  | 13 mg  | 20 mg  |
| Cellulosepulver        | 33 mg  | 36 mg  | 27 mg  | 26 mg  |
| Magnesiumstearat       | 4 mg   | 4 mg   | 4 mg   | 4 mg   |
| Gallopamil             | 100 mg | 100 mg | 100 mg | - mg   |
| Verapamil              | - mg   | - mg   | - mg   | 450 mg |
|                        | 400 mg | 400 mg | 400 mg | 700 mg |

## Ansprüche

1. Gegenstand der Erfindung ist eine feste Arzneimittelzubereitung auf Alginatbasis mit protrahierender Wirkung, dadurch gekennzeichnet, daß sie als Alginat eine Mischung aus einem Alkalialginat oder Polyethylenglykolalginat und einem Alkali-Erdalkali-Alginat enthält.

2. Verfahren zur Herstellung einer Arzneimittelzubereitung auf Alginatbasis mit protrahierender Wirkung, dadurch gekennzeichnet, daß man den Wirkstoff mit einem Alkalialginat, einem Alkali-Erdalkali-Alginat und galenischen Hilfsstoffen mischt und die so erhaltene Mischung zu einer festen Applikationsform verarbeitet.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 188 040 (ABBOTT LABORATORIES LTD) <br> * Seite 2, Zeile 29 - Seite 3, Zeile 34; Seite 4, Zeilen 20-29; Ansprüche 1-10 * | 1,2 | A 61 K 9/22 |
| | --- | | |
| A | FR-A- 985 218 (MEDICAL ALGINATES LTD) <br> * Ansprüche 1-8 * | 1,2 | |
| | --- | | |
| D,A | FR-A-2 118 492 (FIRMA EFEKA FRIEDRICH & KAUFMANN) <br> * Ansprüche 1-8 * | 1,2 | |
| | ----- | | |

RECHERCHIERTE
SACHGEBIETE (Int. Cl.4)

A 61 K

Der vorliegende Recherchenbericht wurde für alle Patentansprüche erstellt.

| Recherchenort | Abschlußdatum der Recherche | Prüfer |
|---|---|---|
| DEN HAAG | 24-09-1987 | TZSCHOPPE,D.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82